# FASCICULE DE BREVET EUROPEEN

(11) **EP 4 103 066 B1**
(45) Date de publication et mention de la délivrance du brevet: **03.04.2024**
(21) Numéro de dépôt: 21705278.6
(22) Date de dépôt: 12.02.2021
(51) Int. Cl.: A61B 17/04, A61B 17/06

(54) **DISPOSITIF D'ANCRAGE POUR LA RÉPARATION DES TISSUS MOUS**
VERANKERUNGSVORRICHTUNG FÜR WEICHGEWEBEREPARATUR
ANCHORING DEVICE FOR SOFT TISSUE REPAIR

(30) Priorité: 14.02.2020 FR 2001485
(43) Date de publication de la demande: 21.12.2022
(73) Titulaire: Teknimed, 65500 Vic-en-Bigorre (FR)
(72) Inventeur: GONCALVES, Stéphane, 31130 Balma (FR)
(74) Mandataire: Argyma
(86) Numéro de dépôt international: PCT/IB2021/051167
(87) Numéro de publication internationale: WO 2021/161236

(56) Documents cités:
- EP-A1- 2 572 650
- EP-A1- 2 662 030
- WO-A1-2019/023205
- WO-A1-2019/032797
- US-A1- 2014 277 133
- US-A1- 2018 344 310
- US-B2- 9 826 971
- US-B2- 9 974 534

## Description

La présente invention appartient au domaine du matériel chirurgical dédié à la réparation des tissus mous et concerne plus particulièrement des dispositifs implantables pour la fixation d'un tissu à une structure osseuse.

Elle a pour objet un dispositif comprenant une tresse textile et des fils de suture agencés pour former un système d'ancrage souple, dont une configuration déployée permet l'introduction dans un trou d'un os, et une configuration comprimée assure l'ancrage dans le trou et la fixation d'un tissu à l'os.

On sait qu'un tendon est une structure formée d'un faisceau de fibres de collagène, reliant un muscle à un os. Bien qu'ils possèdent une forte résistance, les tendons sont sujets à un risque de déchirure, soit à la suite d'un traumatisme, soit du fait de leur dégénérescence. La première situation est plus répandue chez les personnes ayant des professions physiques et chez les sportifs, tandis que la seconde concerne davantage des personnes avancées en âge, souvent de plus de 50 ans. En effet, les mouvements et les efforts répétitifs comme l'entraînement chez les sportifs de haut niveau ou propres à certains métiers ou activités manuelles peuvent induire diverses lésions comme des tendinites et une fragilisation du tendon qui peut se déchirer ou se rompre.

Chaque fois que c'est possible, une intervention chirurgicale est préconisée pour rattacher le tendon déchiré à l'os. Une première technique repose sur un traitement par tunnels transosseux qui vise à rattacher les tendons déchirés à l'os en passant des fils à travers l'os pour fixer les tendons. Une seconde technique consiste en une réparation par ancres de suture, au cours de laquelle le tendon est suturé directement sur l'os à l'aide d'un dispositif comprenant un élément d'ancrage associé à un ou plusieurs fils de suture. L'élément d'ancrage va maintenir le dispositif dans l'os tandis que les fils seront attachés à l'extrémité du tendon à refixer. Cette technique est à la fois très simple à réaliser, avec entre autres une durée d'opération plus courte, et peu invasive avec moins de lésions des tissus mous, ce qui accélère le temps de récupération et de guérison. D'autres types de tissus sont concernés par ce type de réparation, notamment les ligaments.

Différents dispositifs d'ancrage peuvent être utilisés. On connait des ancres de suture rigides, en métal ou en matériaux polymères résorbables ou non. Celles-ci présentent l'inconvénient d'occuper un volume important qui impose de retirer de la matière osseuse, fragilisant de fait la structure et oblige à limiter le nombre de points d'insertion. La fixation en est amoindrie. Pour remédier à cet inconvénient, on peut utiliser des ancres souples et déformables. Le principe est d'introduire l'ancre à l'aide d'un inserteur dans un trou préparé dans l'os (ou réalisé à l'aide de l'inserteur) puis de tirer sur les brins des fils de suture de sorte que l'ancre se plisse ou se recourbe en formant un amas volumineux qui se bloque contre la paroi corticale de l'os. Il ne reste plus alors qu'à attacher les fils de suture au tendon. Ces ancres sont généralement faites en matériau synthétique, qui peut être par exemple sous la forme d'une bande textile, ou encore d'un cordon tressé plein ou tubulaire.

La façon dont l'ancre et des fils sont associés est primordiale pour obtenir une fixation solide et fiable, qui soit facile et rapide à implanter. Il est en particulier nécessaire que le dispositif ait une configuration initialement étirée de faible diamètre, apte à être installée aisément sur un inserteur et insérée dans l'os. Il est également indispensable que l'ancre se déforme sans difficulté une fois placée dans le trou de l'os sous l'effet de la traction des fils, en acquérant un volume conséquent pour rester solidement bloquée lorsqu'elle est installée. En effet, la capacité de déformation en largeur de l'ancre est primordiale pour assurer une bonne résistance à l'arrachement.

De nombreux modèles d'ancres souples expansibles ont été proposés. Par exemple, US 9826971 décrit un dispositif comprenant une bande souple en fibre textile et au moins un fil de suture passant à travers la bande en plusieurs points, de bout en bout, les extrémités du fil étant libres, avec ou sans noeud. La bande souple se plisse quand elle est mise en place. La demande WO 2019 032797 décrit un dispositif d'ancrage similaire, mais comprenant en plus un élément en matériau spongieux tel qu'un tissu éponge en fibre de cellulose qui augmente de volume quand il est en contact avec un activateur tel que de l'eau. Dans US 9974534, une ancre souple ayant une structure tubulaire creuse est faite d'un matériau intissé. Un fil de suture passe à l'intérieur du tube de l'ancre et en ressort à proximité des extrémités, l'ancre étant pliée en deux et implantée dans cette configuration pliée. Au contraire US 2014 277133 propose d'utiliser un implant d'ancrage tubulaire rectiligne, un fil de suture circulant à travers la paroi de l'implant d'une extrémité à l'autre en aller et retour. Dans EP 2662030, une ancre tressée configurée en V pour former deux bras joints par une pointe, est associée avec un fil de suture qui circule d'une extrémité de la tresse jusqu'à la pointe puis de la pointe jusqu'à l'autre extrémité en passant à travers les mailles de la tresse en plusieurs points. Le fil comporte un collet permettant de serrer l'assemblage après installation. EP 2572650 décrit une ancre de suture à tube pliant constituée d'un tube (cylindre) pourvu d'ouvertures/trous pour permettre le passage d'un fil flexible. Lorsque le tube est inséré dans un tunnel osseux/une cavité et qu'une tension est appliquée, le tube se plie et se loge dans le tunnel osseux/la cavité.

WO 2019023205A1 décrit une ancre de suture contenant deux sections une suture à ancrer et un corps d'ancre, qui forme une partie de l'ancre qui peut augmenter en largeur, épaisseur et diamètre et rétrécir en longueur dans le cadre du déploiement.

Toutefois, ces dispositifs souffrent de divers inconvénients en relation avec la fixation entre le fil de suture et l'ancre, avec la fixation de l'ancre à l'os, ou d'autres inconvénients découlant de la configuration de l'assemblage, qui n'offrent pas des conditions optimales pour le chirurgien chargé de l'implantation et finalement pour le patient. Il existe ainsi un besoin de disposer d'un système de fixation supportant d'être mis en tension autant que nécessaire lorsqu'il est implanté dans l'os. Est recherché un assemblage constitué de matériaux souples, qui puisse être aisément introduit dans l'os et ensuite serré par une simple traction des fils de suture.

La présente invention a pour objectif de remédier à ces inconvénients en proposant un dispositif de réparation des tissus mous reposant sur un assemblage original d'une ancre textile avec des fils de suture, qui offre des performances optimales en ce qui concerne les propriétés mécaniques de résistance à la traction, la facilité d'implantation pour le praticien, et un retrait minimum de matière osseuse pour ne pas fragiliser la fixation. Selon l'invention proposée, le dispositif est dans une configuration initiale étirée qui est fine et donc facile à insérer, mais est dotée d'une capacité d'expansion élevée en largeur de façon à bloquer le dispositif textile par appui sur la paroi intérieure de la corticale de l'os recevant l'implant. De manière originale, l'expansion du dispositif est obtenue par l'effet de deux actions conjuguées, à savoir d'une part le repliement de l'ancre en plusieurs boucles agglomérées, et d'autre part le gonflement latéral du tressage de l'ancre. Ce faisant, les fils de suture peuvent coulisser sans se coincer tout le long de l'ancre ce qui s'avère utile pour la mise en place de l'implant, en particulier lors de la réalisation des noeuds chirurgicaux. Ce résultat est obtenu grâce à l'association d'une ancre souple constituée d'une tresse en matériau textile avec des fils de suture traversant le matériau en plusieurs points.

Plus précisément, l'invention a pour objet un dispositif chirurgical pour la réparation des tissus mous, apte à être inséré dans un trou pratiqué dans un os dans une configuration initiale déployée et à adopter une configuration finale comprimée après insertion, le dispositif comprenant, dans sa configuration initiale :
- une ancre souple constituée d'une tresse de fibres textiles, qui est incurvée en arceau en formant un premier et un second bras s'étendant en direction distale à partir d'une zone sommitale,
- au moins un fil de suture dont une partie médiane sépare un premier et un second brins longs ayant chacun une extrémité libre, la partie médiane du fil étant placée entre le premier et le second bras à proximité de la zone sommitale de l'ancre, et les deux brins du fil traversant la tresse transversalement de part en part en plusieurs sites,
   - le premier brin traversant la tresse alternativement en deux sites voisins du premier bras et en deux sites voisins du second bras, les sites traversés successivement sur un même bras étant de plus en plus éloignés de la zone sommitale,
   - le second brin traversant la tresse en au moins deux sites du second bras, les sites traversés successivement étant de plus en plus éloignés de la zone sommitale.

Le dispositif selon l'invention est décrit ici et dans la suite de l'exposé dans sa configuration initiale déployée, c'est-à-dire étalée ou développée dans toute son extension, telle qu'elle se présente à un chirurgien souhaitant procéder à la réparation d'un tissu (tendon, ligament ou autre) par accrochage à un os. La configuration comprimée qu'elle peut adopter une fois mise en place sera détaillée plus loin.

L'ancre est réalisée sous la forme d'une tresse de fibres textiles, c'est-à-dire de fibres susceptibles d'être tressées. Par tresse, on entend un type de tressage dans lequel toutes les fibres sont entrelacées les unes aux autres à tour de rôle, selon une direction relative en diagonale, par opposition à un tressage trame sur chaine. Différents modes de tressage sont possibles. On trouve par exemple des tresses plates et des tresses rondes, pleines ou tubulaires, réalisées avec un nombre variable de fibres. Selon l'invention, les fibres sont souples et tressées avec une tension modérée de sorte que l'ancre est elle-même souple. De ce fait, elle peut être pliée en arceau et elle offre un passage aisé entre ses fibres pour qu'un fil de suture puisse la traverser. En outre et de façon essentielle, elle peut se déformer pour adopter une configuration comprimée et assurer sa fonction d'ancrage.

Dans sa configuration initiale, l'ancre est incurvée en arceau en formant deux bras qui s'étendent en direction distale à partir d'une pointe ou zone sommitale et qui délimitent visuellement un espace intérieur et un espace extérieur de l'arceau. La pointe de l'arceau pourra être engagée sur un outil inserteur pour pousser le dispositif selon son axe dans l'os récepteur. Un fil de suture au moins circule sur la tresse de l'ancre. Dans une variante qui sera décrite plus loin, le dispositif peut comporter un second fil de suture. Les fils de suture sont associés à l'ancre par passage à travers la tresse en différents sites, qui sont généralement des interstices existant entre les fibres de la tresse. Il est donc inutile de percer l'ancre aux endroits où le fil de suture doit passer.

Ledit au moins un fil de suture est enfilé sur l'ancre en passant à travers la tresse à proximité de la zone sommitale. La partie du fil de suture qui se trouve entre les bras de l'ancre est désignée comme étant la partie médiane, à partir de laquelle s'étendent deux brins longs. On fait en sorte que les deux brins soient de longueur comparable de manière à offrir deux extrémités libres faciles à manipuler par le chirurgien. On comprend cependant que dans la mesure où le fil de suture coulisse sur la tresse, la partie médiane n'est pas strictement définie en tant que telle, mais correspond à un segment du fil occupant l'emplacement situé entre les deux bras, à faible distance de la pointe de l'ancre.

Les deux brins du fil de suture traversent la tresse transversalement de part en part en plusieurs sites, chacun selon un schéma particulier. Quel que soit le mode de tressage de l'ancre retenu, les brins du fil de suture traversent la tresse en totalité sensiblement perpendiculairement à son axe principal, sans sinuer dans l'entrelacs des mailles.

Le premier des deux brins traverse la tresse en deux sites voisins l'un de l'autre, en passant alternativement du premier bras au second bras, les sites traversés successivement sur un même bras étant de plus en plus éloignés de la zone sommitale. Le passage alternatif du premier au second bras indique expressément une alternance, c'est-à-dire une succession répétée dans l'espace qui fait réapparaitre tour à tour, dans un ordre régulier, un motif d'une série. En l'espèce, il s'agit ici d'une alternance des paires de sites traversés par le premier brin. Chaque passage dans deux sites voisins d'un bras correspond à une traversée vers l'extérieur de l'arceau suivie d'un retour vers l'intérieur entre les bras de la tresse. Les paires de sites voisins sont progressivement de plus en plus éloignées de la pointe de l'ancre. Elles peuvent être totalement décalées de sorte que chaque site est plus loin de la pointe que le précédent, le fil de suture traversant l'espace intérieur en diagonale, ou bien l'éloignement peut se faire uniquement entre deux sites voisins d'une même paire auquel cas le fil de suture traverse l'espace intérieur perpendiculairement à l'axe de l'ancre.

Le second des deux brins traverse la tresse en au moins deux sites du second bras, les sites traversés successivement étant de plus en plus éloignés de la zone sommitale. Ce brin traverse donc toujours le même bras de l'ancre, le premier passage se faisant vers l'extérieur de la tresse, puis vers l'intérieur, en un site plus éloigné de la pointe de l'ancre, et ainsi de suite.

Selon une caractéristique du dispositif objet de la présente invention, le premier brin traverse la tresse en 2n+1 paires de sites voisins, alternativement du premier bras et du second bras, n étant égal à 1 ou 2. En d'autres termes, le premier brin traverse trois paires de sites voisins ou cinq paires de sites voisins, sur un bras et sur l'autre alternativement. De préférence, trois paires de sites voisins sont traversés, et de préférence aussi, la progression du brin vers l'extrémité des bras de l'ancre se fait par l'espacement entre deux sites voisins, le fil de suture traversant l'espace intérieur de l'arceau perpendiculairement à l'axe de l'ancre. Finalement, le premier brin termine son cheminement à travers la tresse en débouchant dans l'espace intérieur de l'arceau.

Ainsi, selon une caractéristique préférée du dispositif objet de la présente invention, le premier brin traverse la tresse en deux sites voisins du premier bras, en deux sites voisins du second bras, et en deux sites voisins du premier bras, successivement et dans cet ordre, deux sites traversés successivement sur le premier et le second bras étant à une distance sensiblement identique de la zone sommitale.

Selon une autre caractéristique préférée du dispositif objet de la présente invention, le second brin du fil de suture traverse la tresse en deux sites du second bras, l'un à proximité de la zone sommitale et l'autre à proximité de la partie distale dudit second bras. De la sorte, le second brin termine son cheminement à travers la tresse en débouchant dans l'espace intérieur de l'arceau.

Selon une caractéristique préférée du dispositif objet de l'invention, les sites du second bras traversés par le premier brin sont situés entre les deux sites dudit second bras traversés par le second brin. On peut dire de façon imagée que le second brin enjambe le premier brin quand il passe à l'extérieur de l'arceau.

Dans un mode de réalisation préféré du dispositif selon l'invention, l'extrémité libre du premier brin et l'extrémité libre du second brin débouchent chacune d'un site situé à la partie distale d'un des bras de l'ancre dans l'espace compris entre les deux bras, de sorte que les extrémités distales des premier et second bras forment des ailettes adoptant spontanément une orientation divergente par rapport à l'axe de l'ancre. Ces ailettes divergentes ont tendance à s'écarter davantage de part et d'autre lorsque le fil de suture est mis en tension et contribuent ainsi à mieux bloquer l'ancre contre la paroi de l'os récepteur.

De préférence, pour favoriser ce blocage, les extrémités distales des premier et second bras formant les ailettes sont rigidifiées par themodurcissement. On peut par exemple soumettre les extrémités de la tresse, avant l'assemblage du dispositif, à une température comprise par exemple entre 150°C et 400°C en fonction de la matière textile utilisée.

Le dispositif tel que décrit ci-dessus peut être introduit dans un trou d'un os récepteur, en laissant les brins du fil de suture libres à l'extérieur. Une traction sur les brins va lui faire acquérir une configuration comprimée : la tresse se rétracte sur elle-même en s'élargissant et en formant un amas globuleux de boucles, tandis que les ailettes s'écartent, l'ensemble prenant appui dans le trou et contre la paroi corticale de l'os récepteur. L'assemblage de la tresse et du fil de suture constitue ainsi un dispositif d'ancrage, mais c'est la tresse textile qui joue le rôle d'ancre à proprement parler du fait de sa déformation sous l'action de la traction exercée sur les brins du fil de suture, l'extrémité libre des brins étant fixée au tissu à solidariser avec l'os.

Dans un mode de réalisation particulier de l'invention, un second fil de suture est enfilé sur l'ancre en passant à travers la tresse en deux sites voisins entre eux, au niveau de la zone sommitale. Le fil traverse les deux sites depuis l'extérieur de l'arceau, de sorte qu'un segment du fil coiffe la zone sommitale de la tresse, ce segment étant désigné comme la partie médiane du second fil, à partir de laquelle s'étendent deux brins longs. On fait en sorte que les deux brins soient de longueur comparable de manière à offrir deux extrémités libres faciles à manipuler par le chirurgien. On comprend cependant que dans la mesure où le fil de suture coulisse sur la tresse, la partie médiane n'est pas strictement définie en tant que telle, mais correspond à un segment du fil occupant la pointe de l'ancre. Les deux brins s'étendent ensuite entre les bras de l'ancre.

Ainsi, conformément à une variante d'exécution du dispositif selon l'invention, celui-ci comprend un second fil de suture dont une partie médiane sépare un premier et un second brins longs chacun ayant une extrémité libre, lesdits brins traversant la tresse transversalement de part en part en deux sites voisins entre eux de la zone sommitale, de sorte que ladite partie médiane coiffe la zone sommitale et que lesdits premier et second brins s'étendent entre les bras de l'ancre.

Selon une caractéristique préférée du dispositif objet de l'invention, les brins du second fil de suture sont entrelacés avec les portions du premier fil de suture circulant transversalement entre les sites du premier et du second bras. Par exemple, les brins du second fil passent successivement au-dessus puis au-dessous des portions transversales du premier fil de suture, les deux brins pouvant sinuer simultanément en phase, ou au contraire séparément de manière décalée. Les brins du second fil de suture restent ainsi orientés entre les bras de l'ancre, en bonne position pour la mise en oeuvre du dispositif par le chirurgien. Lorsque les deux brins sinuent simultanément, les portions transversales du premier fil de suture sont légèrement repoussées en s'incurvant autour des deux brins, de sorte qu'un passage apparait le long des deux brins, facilitant l'introduction d'un outil inserteur jusqu'à la zone sommitale de l'ancre.

Lorsque le chirurgien installe l'implant, il va tirer les deux brins du premier fil de suture pour plisser les bras de la tresse, mais aussi les deux brins du second fil de suture ce qui va amener la pointe de l'ancre vers la corticale et imposer son agglomération conjointement avec les plissements des bras pour créer un agglomérat plus volumineux, assurant une fixation renforcée.

L'obtention d'un amas volumineux est également assurée par le choix du type de tresse constituant l'ancre. C'est pourquoi, conformément à une caractéristique préférée du dispositif selon l'invention, l'ancre est constituée d'une gaine tubulaire tressée, prise dans une conformation aplatie selon son axe longitudinal. La gaine aplatie forme donc deux épaisseurs de tressage superposées. Les tresses tubulaires sont connues pour la fabrication d'ancres chirurgicales, mais elles sont communément utilisées pour faire circuler un ou plusieurs fils de suture dans la lumière centrale, contrairement au présent dispositif. Or, de manière inattendue, il est apparu que les fibres d'une tresse tubulaire pouvaient en se déformant, conférer un effet de gonflement plus marqué quand un fil traverse la tresse dans toute son épaisseur. La tresse tubulaire s'aplatit en une double couche nattée qui va se comprimer selon deux processus. D'une part, la tresse se coude en formant des plis plus ou moins ordonnés, et d'autre part la tresse elle-même s'évase latéralement donnant un volume supplémentaire à l'ancre comprimée. Finalement, on obtient une excellente capacité de gonflement, d'expansion en épaisseur et largeur de l'ancre. Ceci permet d'utiliser des ancres de taille moindre qui assurent une tenue mécanique optimale et donc une fixation sécurisée.

En pratique, il est avantageux de réaliser l'assemblage de l'ancre avec le ou les fils de suture, à partir d'une tresse préalablement aplatie, ce qu'on peut aisément faire par un traitement thermique doux, par exemple de l'ordre de 150°C. Il s'avère que cette disposition améliore encore le dispositif, en favorisant une certaine rigidité de la tresse et en permettant le passage des fils de suture à travers les mailles de la tresse et leur coulissement. Ainsi, de préférence, la gaine est aplatie à chaud avant découpe à une longueur choisie, comprise entre 2 cm et 5 cm.

La gaine est formée d'une pluralité de fibres textiles. Les paramètres du tressage sont adaptés en fonction du nombre de fils et de leur titrage, pour obtenir une gaine modérément compacte, permettant le passage d'une aiguille à épissure entre les mailles tout en conservant un pouvoir de gonflement de la tresse élevé. La gaine peut être formée par exemple, de 4 à 6 fibres textiles titrant de 20 dtex à 200 dtex (dtex : masse en g de 10 000 m de fil). L'homme de l'art sait modifier les paramètres de tressage, tels que le point ou le nombre de motifs par unité de longueur à cette fin en jouant sur les réglages du métier à tresser.

En ce qui concerne les fils de suture, ils peuvent être commodément choisis parmi ceux déjà homologués par exemple par la Pharmacopée des États-Unis (USP), de type monofils ou tressés, résorbables ou non. Les fils tressés présentent l'avantage d'avoir un coefficient de friction plus élevé qui sécurise les noeuds. Des fils de diamètre compris entre 0,4 mm et 0,7 mm par exemple, conviennent. On précise que dans tous les modes de réalisation de l'invention présentés ci-dessus, ledit au moins un fil de suture et le second fil de suture s'il est présent, sont coulissants sur toute leur longueur par rapport à la tresse de l'ancre.

De nombreux matériaux peuvent être utilisés pour fabriquer le dispositif selon l'invention, parmi lesquels on trouve tout naturellement les polymères de synthèse convenant aux dispositifs médicaux et plus spécifiquement à une utilisation chirurgicale. Pourront être employés en particulier les polyéthylènes, qu'il s'agisse de polyéthylènes à très haut poids moléculaire (ou UHMWPE pour ultra-high-molecular-weight polyethylene), de polyéthylènes à très faible poids moléculaire (ULMWPE pour ultra-light-molecular-weight polyethylene), de polyéthylènes haute densité (HTPE pour high-tenacity polyethylene) ou des polyéthylènes standard (PE). Pourront également être employés des polytéréphtalates d'éthylène (PET pour polyethylene terephtalate) ou encore des acides polylactiques (PLA pour polylactic acid), ces derniers étant résorbables. L'homme de l'art connait ces matériaux et peut se les procurer auprès des fabricants en fonction des spécifications qu'il définit.

Ainsi, selon une caractéristique de l'invention, le dispositif peut être réalisé en matériau polymère, resorbable ou non résorbable, identique ou différent pour l'ancre, pour ledit au moins un fil de suture et pour le second fil de suture s'il est présent. De manière avantageuse, ledit matériau polymère peut être choisi parmi les polyéthylènes, les polyéthylènes téréphtalates, les acides polylactiques.

Le dispositif chirurgical décrit ci-dessus dans sa configuration initiale étirée doit être placé sur un outil inserteur et enfoncé soit directement dans un os récepteur, soit dans un trou préalablement foré ou percé à l'aide d'un pointeau taraud, pour introduire l'ancre en totalité dans le trou, en laissant les brins du fil ou des fils de suture libres à l'extérieur. Cette configuration a une première longueur (hors brins libres) et une première largeur suffisamment réduites pour permettre l'introduction du dispositif dans un trou d'un os lors d'une intervention chirurgicale chez l'humain ou l'animal, avec un minimum d'extraction de matière osseuse. L'inserteur une fois retiré, le chirurgien va tirer sur les brins pour provoquer une déformation de la tresse. Celle-ci se rétracte sur elle-même en s'élargissant et en formant un amas globuleux de boucles tandis que les ailettes s'écartent. Le dispositif acquiert finalement une configuration comprimée : les bras de l'ancre sont repliés et la tresse est tassée selon son axe. Cette configuration a une deuxième longueur inférieure à la première longueur et une deuxième largeur supérieure à la première largeur. L'ensemble réalise un ancrage prenant appui dans le trou et contre la paroi corticale intérieure de l'os récepteur.

Ainsi, selon une caractéristique du dispositif chirurgical pour la réparation des tissus mous objet de la présente invention, celui-ci possède :
- une configuration initiale déployée dans laquelle les bras de l'ancre sont étendus et la tresse est étirée selon son axe, laquelle configuration a une première longueur et une première largeur autorisant l'introduction du dispositif dans un trou d'un os humain ou animal, et
- une configuration finale compressée obtenue par traction des brins dudit au moins un fil de suture, dans laquelle les bras de l'ancre sont repliés et la tresse est tassée selon son axe, laquelle configuration a une deuxième longueur inférieure à la première longueur et une deuxième largeur supérieure à la première largeur, conférant au dispositif sa conformation d'ancrage dans le trou.

L'assemblage du dispositif selon l'invention assure une bonne répartition des fils et un encombrement équilibré. L'expansion latérale est importante, le diamètre étant de l'ordre du double entre la configuration initiale et la configuration finale (passant par exemple d'environ 5 mm à environ 10 mm). La sécurité de la fixation est assurée à la fois par la présence des ailettes et par la double épaisseur de la tresse qui évite un possible déchirement. Le coulissement des brins est conservé y compris après serrage. Les propriétés mécaniques de résistance à la traction ont été testées avec succès. Il convient également de relever que l'assemblage du dispositif ne soulève pas de difficultés particulières, ce qui est indispensable pour une fabrication industrielle.

Un dispositif selon l'invention est particulièrement utile pour la réparation des tissus mous devant être fixés à un os support des membres supérieurs (épaule, coude, poignet, main) comme des membres inférieurs (hanche, genou, cheville, pied). Cette réparation peut consister à refixer le tissu après section ou arrachement, à le renforcer après une lésion ou à le reconstruire. Au niveau de l'épaule, les interventions peuvent concerner par exemple la réparation de la coiffe des rotateurs, des lésions du labrum, des capsules, de l'instabilité de l'épaule antérieure, des lésions de Bankart, des deltoïdes, la réparation acromioclaviculaire, la reconstruction capsulolabrale, ... On peut utiliser également le dispositif pour la ténodèse du biceps, pour la remise en place du tendon du biceps au coude, pour la reconstruction du tendon du biceps, du ligament collatéral ulnaire et radial, pour la réparation de l'épicondylite latérale, ... Le dispositif s'appliquera aussi à la réparation du milieu du pied, de l'avant-pied et de la cheville, à la reconstruction du tendon d'Achille, de l'hallux valgus, ainsi qu'aux réparations de la stabilisation latérale ou médiale, ...

La présente invention sera mieux comprise, et des détails en relevant apparaîtront, à la lumière de la description qui va être faite de différentes variantes de réalisation, en relation avec les figures annexées, dans lesquelles :
La Fig. 1 représente un dispositif conforme à l'invention dans lequel une ancre et un fil de suture sont assemblés selon un premier mode de réalisation.
La Fig. 2 représente un dispositif conforme à l'invention dans lequel une ancre et un fil de suture sont assemblés selon un deuxième mode de réalisation.
La Fig. 3 représente un dispositif conforme à l'invention dans lequel une ancre et un fil de suture sont assemblés selon un troisième mode de réalisation.
La Fig. 4 représente un dispositif conforme à l'invention dans lequel une ancre et un fil de suture sont assemblés selon un quatrième mode de réalisation.
La Fig. 5 représente un dispositif conforme à l'invention dans lequel une ancre et deux fils de suture sont assemblés.
La Fig.6 représente le même dispositif après mise en place sur un outil inserteur pour son implantation dans un os récepteur.

### EXEMPLE 1

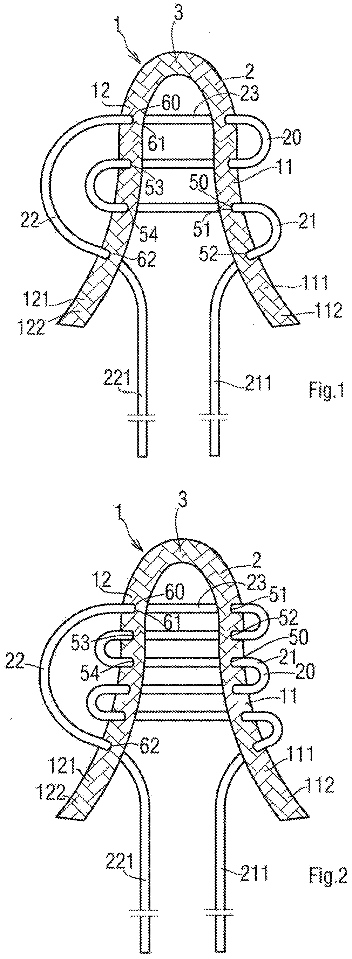

Sur les Fig. 1 à 4, est représenté en configuration déployée (c'est-à-dire étirée, avant installation), un dispositif chirurgical dans une première variante de réalisation, qui comprend une ancre 1 assemblée avec un fil de suture 20. L'ancre 1 est constituée d'une tresse 2 incurvée en arceau. L'arceau comporte une zone sommitale 3, ou pointe, à partir de laquelle un premier 11 et un second bras 12 s'étendent en direction distale, qui définissent un espace intérieur et un espace extérieur de l'arceau repérable visuellement. La tresse 2 est une gaine tubulaire, réalisée à l'aide de fibres synthétiques, ici en polyéthylène de très haute masse moléculaire. La tresse 2 tubulaire, initialement de section ronde, est aplatie par pressage à chaud, par exemple à une température de 150°C durant 15 s à 30 s, de sorte qu'elle comporte une double épaisseur de fibres entrelacées, formant deux couches parfaitement superposées et solidaires. Un tressage à six fibres de titrage 50 dtex, avec environ huit motifs par cm permet d'obtenir une rigidité de l'ancre 1 convenable et un écartement des mailles autorisant le passage d'une aiguille épissure pour l'assemblage avec le fil de suture 20. Les extrémités de la tresse 2 peuvent être thermiquement fixées par exemple grâce à une découpe à l'aide d'un couteau chauffant à la longueur voulue.

Le fil de suture 20 est associé à cette ancre en traversant la tresse 2 transversalement de part en part en plusieurs sites 50. On a utilisé ici un fil de suture de type standard, en polyéthylène UHMWPE, de diamètre USP 2 (0,500 mm - 0,599 mm). Il est enfilé à travers les mailles de la tresse 2 qui s'écartent légèrement au passage d'un outil tel qu'une aiguille à épissure, sans qu'il soit nécessaire de percer une quelconque perforation. Le fil de suture 20 est d'abord piqué dans la tresse en deux sites 50, l'un sur le premier bras 11, l'autre sur le second bras 12, à proximité de la zone sommitale 3, par exemple à 5 mm de la pointe extrême de l'arceau. Le passage se fait de l'intérieur vers l'extérieur de l'arceau, de sorte qu'une portion du fil de suture 20 se trouve placée entre le premier et le second bras à proximité de la zone sommitale 3 de l'ancre. Cette portion, appelée partie médiane 23, sépare un premier brin 21 et un second brin 22 du fil de suture 20. Chaque brin traverse la tresse 2 transversalement de part en part en plusieurs sites 50, puis se termine par une extrémité libre 211 et 221, d'une longueur de plusieurs dizaines de cm (par exemple de 20 cm à 70 cm) permettant la ligature des tissus mous de manière commode. Ces longueurs et positions sont données pour un dispositif dans l'état où le chirurgien le reçoit, mais elles peuvent être modifiées lors de sa manipulation, notamment par coulissement ou glissement du fil de suture 20 par rapport à la tresse 2.

L'agencement du fil de suture 20 par rapport à l'ancre 1 répond à plusieurs critères, avec des points communs et des variations possibles concernant chacun des deux brins 21, 22. Ils ont en commun de traverser la tresse 2 chacun un nombre pair de fois, en progressant des environs de la zone sommitale vers la partie distale des bras 11, 12. Puisqu'ils débutent leur circulation sur la tresse en la traversant vers l'extérieur de l'arceau, ils la terminent toujours en débouchant vers l'intérieur de l'arceau. Les brins libres 211, 221 du fil de suture 20 se trouvent ainsi situées entre les extrémités distales des deux bras 11, 12. Ces extrémités distales adoptent spontanément une orientation divergente par rapport à l'axe de l'ancre 1, formant deux ailettes 112, 122, longues par exemple d'environ 5 mm. Ces dernières ont tendance à s'écarter de l'axe de l'ancre d'autant plus que les brins 21, 22 sont tendus. Elles remplissent ainsi une fonction d'accrochage sur la cloison corticale de l'os récepteur de l'implant, permettant une meilleure stabilisation *in situ* et renforçant la résistance à l'arrachement. Elles peuvent subir un traitement en vue de les durcir davantage (par pressage à chaud, imprégnation d'un polymère, enduction de colle, ...).

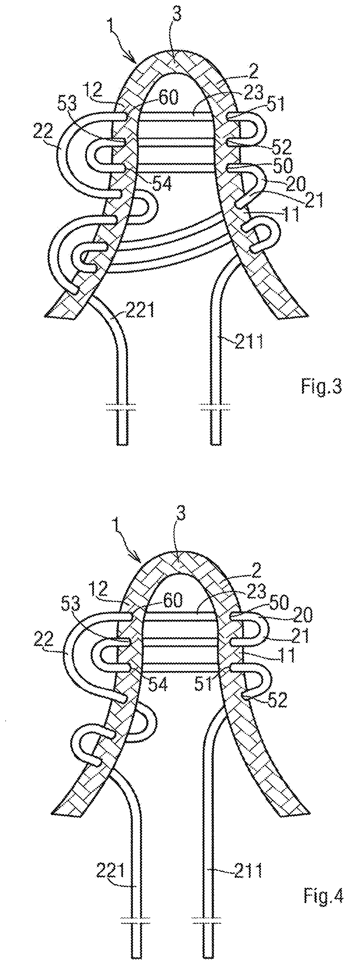

Chaque brin 21, 22 progresse sur la tresse selon un cheminement différent. Le premier brin 21 pique dans la tresse 2 en deux sites 50 voisins l'un de l'autre, situés sur un bras, puis va piquer dans la tresse 2 en deux sites 50 voisins l'un de l'autre, situés sur l'autre bras, à plusieurs reprises. Plus précisément, il traverse d'abord deux sites voisins 51, 52 du premier bras 11, puis deux sites voisins 53, 54 du second bras 12, puis retour vers deux sites voisins 51, 52 du premier bras 11, et ainsi de suite, toujours vers l'extérieur puis vers l'intérieur de l'arceau et en progressant des environs de la zone sommitale vers l'extrémité des bras 11, 12, jusqu'à la partie distale 111 du premier bras 11. Selon une variante d'exécution représentée à la Fig. 1, le premier brin 21 traverse la tresse 2 en trois paires de sites voisins, alternativement du premier bras 11 (deux paires de sites 51, 52) et du second bras 12 (une paire de sites 53, 54). Il en est de même selon la variante représentée à la Fig. 4. Selon des variantes d'exécution représentées aux Fig. 2 et 3, le premier brin 21 traverse la tresse 2 en cinq paires de sites voisins, alternativement du premier bras 11 (trois paires de sites 51, 52) et du second bras 12 (deux paires de sites 53, 54). De préférence, les sites 50 d'un même bras sont sensiblement équidistants. La distance séparant deux sites voisins 51, 52 ou 53, 54 correspond à l'avancement progressif du brin de la zone sommitale 3 vers la partie distale de chaque bras. Par contre, comme illustré aux Fig. 1, 2 et 4, deux sites 50 traversés successivement sur le premier bras 11 et le second bras 12 (et inversement sur le second bras 12 et le premier bras 11) sont à une distance sensiblement identique de la zone sommitale 3. Comme illustré à la Fig. 3, cette distance peut être plus importante. Quoi qu'il en soit, le cheminement du premier brin 21 se termine à la partie distale du premier bras 11, vers l'intérieur de l'arceau.

Le second brin 22 du fil de suture 20 pique quant à lui dans la tresse 2 en deux sites 60 situés sur le second bras 12, à plusieurs reprises. Plus précisément, il traverse deux sites 60 du second bras 12, à deux ou quatre reprises toujours vers l'extérieur puis vers l'intérieur de l'arceau et en progressant des environs de la zone sommitale vers la partie distale du second bras 12. Selon une variante d'exécution représentée à la Fig. 1, le second brin 22 traverse la tresse 2 en deux sites du second bras 12, à savoir un site 61 à proximité de la zone sommitale 3 et l'autre site 62 à proximité de la partie distale 121 dudit second bras. Il en est de même selon la variante représentée à la Fig. 2. Dans ces cas, il est préférable les deux sites 61, 62 encadrent les sites 53, 54 du second bras 12 traversés par le premier brin 21, c'est-à-dire que les sites 53, 54 traversés par le premier brin 21 soient situés entre les deux sites 61, 62 traversés par le second brin 22. Selon des variantes d'exécution représentées aux Fig. 3 et 4, le second brin 22 traverse la tresse 2 en quatre sites 60, qui peuvent être intercalés ou pas entre les paires de sites 53, 54 traversés par le premier brin 21.

### EXEMPLE 2

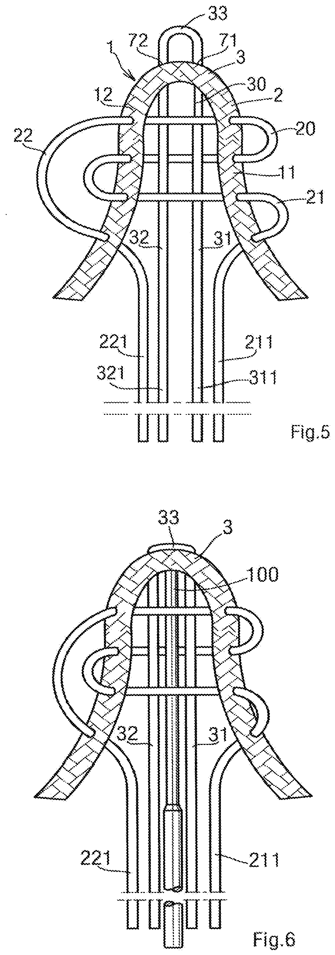

Le dispositif chirurgical dans une deuxième variante de réalisation, peut comprendre une ancre 1 assemblée avec deux fils de suture 20, 30. L'ancre 1 est constituée d'une tresse 2 incurvée en arceau, et le premier fil de suture 20 est associé à cette ancre en traversant la tresse 2 transversalement de part en part en plusieurs sites 50, comme décrit à l'exemple 1 ci-dessus. La Fig. 5 illustre un dispositif en configuration déployée, dans lequel un second fil de suture 30 est associé au montage représenté à la Fig. 1. Ce dispositif peut également être associé avec les autres montages présentés à l'exemple 1.

Le second fil de suture 30 est associé à l'ancre 1 en traversant la tresse 2 transversalement de part en part en deux sites 71, 72 voisins entre eux, situés sur la zone sommitale 3 de l'ancre 1. On a utilisé ici un fil de suture de type standard, en polyéthylène UHMWPE, de diamètre USP 2, d'une couleur permettant de le distinguer facilement du premier fil de suture. Il est enfilé à travers les mailles de la tresse 2 qui s'écartent légèrement au passage d'un outil tel qu'une aiguille à épissure, sans qu'il soit nécessaire de percer une quelconque perforation. Le passage du second fil 30 se fait de l'extérieur vers l'intérieur de l'arceau, de sorte qu'une portion du fil de suture 30 se trouve placée au-dessus de la zone sommitale 3, et contre celle-ci quand le second fil de suture est tendu. Cette portion, appelée partie médiane 33, sépare un premier brin 31 et un second brin 32 du fil de suture 30. Chaque brin traverse la tresse 2 transversalement de part en part aux sites 71, 72, qui s'étend entre les bras 11, 12 de l'ancre 1, puis se termine par une extrémité libre 311 et 321, d'une longueur de plusieurs dizaines de cm (par exemple de 20 cm à 70 cm) permettant la ligature des tissus mous de manière commode. Ces longueurs et positions sont données pour un dispositif dans l'état où le chirurgien le reçoit, mais elles peuvent être modifiées lors de sa manipulation, notamment par coulissement ou glissement du fil de suture 30 par rapport à la tresse 2. Comme illustré à la Fig. 5, les brins 31, 32 du second fil de suture 30 peuvent être entrelacés avec les portions du premier fil de suture 20 circulant transversalement entre les sites 50 du premier et du second bras 11, 12, en passant alternativement au-dessus et au-dessous. Le cheminement des brins 31, 32 du second fil de suture 30 est avantageusement simultané, de manière à créer un passage et à matérialiser un guidage pour un inserteur 100 jusqu'à la zone sommitale 3, comme illustré à la Fig. 6.

Les essais réalisés sur le dispositif d'ancrage selon l'invention ont montré d'excellents résultats en ce qui concerne les propriétés mécaniques de résistance à la traction lors de leur mise en place. Leurs propriétés d'un point de vue de leur expansion en volume et gonflement ont été testées avec succès au regard du risque d'arrachement. Également, la fonction de coulissage avant et après implantation, indispensable pour la réalisation des noeuds chirurgicaux, est assurée. Leur taille est satisfaisante, aucun excédent de matière n'ayant été observé. Enfin, le montage sur l'inserteur ne soulève pas de difficulté.

## Revendications

1. Dispositif chirurgical pour la réparation des tissus mous, apte à être inséré dans un trou pratiqué dans un os dans une configuration initiale déployée et à adopter une configuration finale comprimée après insertion, le dispositif comprenant dans sa configuration initiale :
- une ancre souple (1) constituée d'une tresse (2) de fibres textiles, qui est incurvée en arceau en formant un premier et un second bras (11, 12) s'étendant en direction distale à partir d'une zone sommitale (3), et
- au moins un fil de suture (20) dont une partie médiane (23) sépare un premier et un second brins (21, 22) longs ayant chacun une extrémité libre (211, 221), la partie médiane (23) du fil (20) étant placée entre le premier et le second bras (11, 12) à proximité de la zone sommitale (3) de l'ancre (1), et les deux brins (21, 22) du fil de suture (20) traversant la tresse (2) transversalement de part en part en plusieurs sites (50),
- le premier brin (21) traversant la tresse (2) alternativement en deux sites voisins (51, 52) du premier bras (11) et en deux sites voisins (53, 54) du second bras (12), les sites traversés successivement sur un même bras étant de plus en plus éloignés de la zone sommitale (3), et
- le second brin (22) traversant la tresse (2) en au moins deux sites (60) du second bras (12), les sites traversés successivement étant de plus en plus éloignés de la zone sommitale (3).

2. Dispositif selon la revendication 1, dans lequel le premier brin (21) traverse la tresse (2) en 2n+1 paires de sites voisins, alternativement du premier bras (11) et du second bras (12), n étant égal à 1 ou 2.

3. Dispositif selon la revendication 1 ou 2, dans lequel le premier brin (21) traverse la tresse (2) en deux sites voisins (51, 52) du premier bras (11), en deux sites voisins (53, 54) du second bras (12), et en deux sites voisins (51, 52) du premier bras (11), successivement et dans cet ordre, deux sites (50) traversés successivement sur ledit premier bras et ledit second bras étant à une distance sensiblement identique de la zone sommitale (3).

4. Dispositif selon l'une des revendications précédentes, dans lequel le second brin (22) du fil de suture (20) traverse la tresse (2) en deux sites (61, 62) du second bras (12), l'un à proximité de la zone sommitale (3) et l'autre à proximité de la partie distale

5. Dispositif selon la revendication précédente, dans lequel les sites (53, 54) du second bras (12) traversés par le premier brin (21) sont situés entre les deux sites (61, 62) dudit second bras traversés par le second brin (22).

6. Dispositif selon l'une des revendications précédentes, dans lequel l'extrémité libre (211) du premier brin (21) et l'extrémité libre (221) du second brin (22) débouchent chacune d'un site (50) situé à la partie distale (111, 121) d'un des bras de l'ancre (1) dans l'espace compris entre les deux bras (11, 12), de sorte que les extrémités distales des premier et second bras (11, 12) forment des ailettes (112, 122) adoptant spontanément une orientation divergente par rapport à l'axe de l'ancre (1).

7. Dispositif selon la revendication précédente, dans lequel les extrémités distales des premier et second bras (11, 12) formant les ailettes (112, 122) sont rigidifiées par themodurcissement.

8. Dispositif selon l'une quelconque des revendications précédentes, comprenant un second fil de suture (30) dont une partie médiane (33) sépare un premier et un second brins (31, 32) longs, chacun ayant une extrémité libre (311, 321), lesdits brins traversant la tresse (2) transversalement de part en part en deux sites (71, 72) voisins entre eux de la zone sommitale (3), de sorte que ladite partie médiane coiffe ladite zone sommitale et que lesdits premier et second brins (31, 32) s'étendent entre les bras (11, 12) de l'ancre (1).

9. Dispositif selon la revendication précédente, dans lequel les brins (31, 32) du second fil de suture (30) sont entrelacés avec les portions du premier fil de suture (20) circulant transversalement entre les sites (50) du premier et du second bras (11, 12).

10. Dispositif selon l'une quelconque des revendications précédentes, dans lequel l'ancre (1) est constituée d'une gaine tubulaire tressée, prise dans une conformation aplatie selon son axe longitudinal.

11. Dispositif selon la revendication précédente, dans lequel la gaine est aplatie à chaud avant découpe à une longueur choisie, comprise entre 2 cm et 5 cm.

12. Dispositif selon l'une des revendications 10 ou 11, dans lequel la gaine est formée de 4 à 6 fibres textiles, de titrage compris entre 20 dtex et 200 dtex, tressées ensemble.

13. Dispositif selon l'une quelconque des revendications précédentes, réalisé en matériau polymère resorbable ou non résorbable, identique ou différent pour l'ancre (1), pour ledit au moins un fil de suture (20) et pour le second fil de suture (30) s'il est présent.

14. Dispositif selon la revendication précédente, dans lequel ledit matériau polymère est choisi parmi les polyéthylènes, les polyéthylènes téréphtalates, les acides polylactiques.

15. Dispositif chirurgical pour la réparation des tissus mous selon l'une quelconque des revendications précédentes, possédant :
- une configuration initiale déployée dans laquelle les bras (11, 12) de l'ancre (1) sont étendus et la tresse (2) est étirée selon son axe, laquelle configuration a une première longueur et une première largeur autorisant l'introduction du dispositif dans un trou d'un os humain ou animal, et
- une configuration finale comprimée obtenue par traction des brins (21, 22) dudit au moins un fil de suture (20), et des brins (31, 32) du second fil de suture (30) s'il est présent, dans laquelle les bras (11, 12) de l'ancre (1) sont repliés et la tresse (2) est tassée selon son axe, laquelle configuration a une deuxième longueur inférieure à la première longueur et une deuxième largeur supérieure à la première largeur, conférant au dispositif sa conformation d'ancrage dans le trou.

## Patentansprüche

1. Chirurgische Vorrichtung zur Wiederherstellung von Weichgewebe, die dazu befähigt ist, in einer anfänglichen ausgefahrenen Konfiguration in ein Loch eingebracht zu werden, welches in einen Knochen gebohrt wurde, und nach dem Einbringen eine komprimierte Endkonfiguration einzunehmen, wobei die Vorrichtung in ihrer anfänglichen Konfiguration Folgendes umfasst:
- ein biegsames Ankerorgan (1), das aus einem Flechtwerk (2) aus Textilfasern besteht, welches bogenförmig gekrümmt ist, wobei es einen ersten und einen zweiten Arm (11, 12) bildet, welche sich ausgehend von einem sommitalen Bereich (3) in distaler Richtung erstrecken, und
- mindestens einen Nähfaden (20), wobei ein mittlerer Abschnitt (23) desselben einen ersten und einen zweiten langen Strang (21, 22) trennt, die jeweils ein freies Ende (211, 221) aufweisen, wobei der mittlere Abschnitt (23) des Fadens (20) zwischen dem ersten und dem zweiten Arm (11, 12) in der Nähe des sommitalen Bereichs (3) des Ankerorgans (1) angeordnet ist und die beiden Stränge (21, 22) des Nähfadens (20) das Flechtwerk (2) an mehreren Stellen (50) vollständig in Querrichtung durchsetzen,
- wobei der erste Strang (21) das Flechtwerk (2) im Wechsel an zwei benachbarten Stellen (51, 52) des ersten Arms (11) und an zwei benachbarten Stellen (53, 54) des zweiten Arm (12) durchsetzt, wobei die Stellen, welche auf ein und demselben Arm nacheinander durchsetzt werden, sich in zunehmender Entfernung vom sommitalen Bereich (3) befinden, und
- wobei der zweite Strang (22) das Flechtwerk (2) an mindestens zwei Stellen (60) des zweiten Arms (12) durchsetzt, wobei die Stellen, welche nacheinander durchsetzt werden, sich in zunehmender Entfernung vom sommitalen Bereich (3) befinden.

2. Vorrichtung nach Anspruch 1, wobei der erste Strang (21) das Flechtwerk (2) an 2n+1 Paaren von benachbarten Stellen im Wechsel auf dem ersten Arm (11) und dem zweiten Arm (12) durchsetzt, wobei n gleich 1 oder 2 ist.

3. Vorrichtung nach Anspruch 1 oder 2, wobei der erste Strang (21) das Flechtwerk (2) an zwei benachbarten Stellen (51, 52) des ersten Arms (11), an zwei benachbarten Stellen (53, 54) des zweiten Arms (2) und an zwei benachbarten Stellen (51, 52) des ersten Arms (11) durchsetzt, und zwar nacheinander in dieser Reihenfolge, wobei zwei Stellen (50), welche nacheinander auf dem ersten Arm und dem zweiten Arm durchsetzt werden, sich in einer im Wesentlichen gleichen Entfernung vom sommitalen Bereich (3) befinden.

4. Vorrichtung nach einem der vorhergehenden Ansprüche, wobei der zweite Strang (22) des Nähfadens (20) das Flechtwerk (2) an zwei Stellen (61, 62) des zweiten Arms (12) durchsetzt, wobei sich eine davon in der Nähe des sommitalen Bereichs (3) und die andere in der Nähe des distalen Bereichs (121) des zweiten Arms befindet.

5. Vorrichtung nach dem vorhergehenden Anspruch, wobei die Stellen (53, 54) des zweiten Arms (12), welche von dem ersten Strang (21) durchsetzt werden, sich zwischen den beiden Stellen (61, 62) des zweiten Arms befinden, welche von dem zweiten Strang (22) durchsetzt werden.

6. Vorrichtung nach einem der vorhergehenden Ansprüche, wobei das freie Ende (211) des ersten Strangs (21) und das freie Ende (221) des zweiten Strangs (22) jeweils aus einer Stelle (50) hervorgehen, die sich am distalen Abschnitt (111, 121) eines der Arme des Ankerorgans (1) im Raum zwischen den beiden Armen (11, 12) befindet, sodass die distalen Enden des ersten und zweiten Arms (11, 12) Flügel (112, 122) bilden, die spontan einer auseinanderlaufende Ausrichtung gegenüber der Achse des Ankerorgans (1) einnehmen.

7. Vorrichtung nach dem vorhergehenden Anspruch, wobei die distalen Enden des ersten und des zweiten Arms (11, 12), welche die Flügel (112, 122) bilden, durch Wärmehärtung versteift werden.

8. Vorrichtung nach einem beliebigen der vorhergehenden Ansprüche, die mindestens einen zweiten Nähfaden (30) umfasst, wobei ein mittlerer Abschnitt (33) desselben einen ersten und einen zweiten langen Strang (31, 32) trennt, welche jeweils ein freies Ende (311, 321) aufweisen, wobei die Stränge das Flechtwerk (2) an zwei zueinander benachbarten Stellen (71, 72) des sommitalen Bereichs (3) vollständig in Querrichtung durchsetzen, sodass der mittlere Abschnitt auf dem sommitalen Bereich sitzt und der erste und der zweite Strang (31, 32) sich zwischen den Armen (11, 12) des Ankerorgans (1) erstrecken.

9. Vorrichtung nach dem vorhergehenden Anspruch, wobei die Stränge (31, 32) des zweiten Nähfadens (30) mit den Teilstücken des ersten Nähfadens (20) verwoben sind, welche in Querrichtung zwischen den Stellen (50) des ersten und des zweiten Arms (11, 12) verlaufen.

10. Vorrichtung nach einem beliebigen der vorhergehenden Ansprüche, wobei das Ankerorgan (1) aus einer geflochtenen röhrenförmigen Umhüllung besteht, die in einer Formgebung herangezogen wird, welche gemäß ihrer Längsachse abgeflacht ist.

11. Vorrichtung nach dem vorhergehenden Anspruch, wobei die Umhüllung in der Hitze abgeflacht wird, bevor sie auf eine gewählte Länge zugeschnitten wird, die im Bereich von 2 cm bis 5 cm liegt.

12. Vorrichtung nach einem der Ansprüche 10 oder 11, wobei die Umhüllung aus 4 bis 6 Textilfasern gebildet ist, deren Feinheit im Bereich von 20 dtex bis 200 dtex liegt, wobei sie miteinander verflochten sind.

13. Vorrichtung nach dem einem beliebigen der vorhergehenden Ansprüche, wobei sie, was den mindestens einen Nähfaden (20) und den zweiten Nähfaden (30) betrifft, aus einem resorbierbaren oder nicht resorbierbaren Polymermaterial hergestellt ist, bei welchem es sich um das gleiche wie für das Ankerorgan (1) oder um ein andersartiges handelt.

14. Vorrichtung nach dem vorhergehenden Anspruch, wobei das Polymermaterial aus den Polyethylenen, den Polyethylenterephthalaten, den Polymilchsäuren ausgewählt ist.

15. Chirurgische Vorrichtung zur Wiederherstellung von Weichgewebe nach einem beliebigen der vorhergehenden Ansprüche, wobei sie Folgendes besitzt:
- eine anfängliche ausgefahrene Konfiguration, in welcher die Arme (11, 12) des Ankerorgans (1) ausgestreckt sind und das Flechtwerk (2) gemäß seiner Achse gestreckt ist, wobei diese Konfiguration eine erste Länge und eine erste Breite hat, die es ermöglichen, dass die Vorrichtung in ein Loch in einem menschlichen oder tierischen Knochen eingebracht wird, und
- eine komprimierte Endkonfiguration, die erhalten wird, indem an den Strängen (21, 22) des mindestens einen Nähfadens (20) und an den Strängen (31, 32) des zweiten Nähfadens (30), sofern ein solcher vorliegt, gezogen wird, wobei die Arme (11, 12) des Ankerorgans (1) angelegt und das Flechtwerk (2) gemäß seiner Achse gestaucht wird, wobei diese Konfiguration eine zweite Länge, welche geringer als die erste Länge ist, und eine zweite Breite hat, welche größer als die erste Länge ist, sodass die Vorrichtung ihre Formgebung zur Verankerung in dem Loch erhält.

## Claims

1. Surgical device for the repair of soft tissues which is capable of being inserted into a hole produced in a bone in an initial deployed configuration and of adopting a final compressed configuration after insertion, the device comprising in its initial configuration:
- a flexible anchor (1) constituted by a braid (2) of textile fibers, which is curved into a hoop to form a first and a second arms (11, 12) extending distally from an apex zone (3), and
- at least one suture filament (20), a median portion (23) of which separating a first and a second long strands (21, 22) each having a free end (211, 221), the median portion (23) of the suture filament (20) being placed between the first and the second arms (11, 12) in the vicinity of the apex zone (3) of the anchor (1), and the two strands (21, 22) of the suture filament (20) passing transversely through the braid (2) from one side to the other at a plurality of sites (50),
- the first strand (21) passing through the braid (2) alternately at two adjacent sites (51, 52) of the first arm (11) and at two adjacent sites (53, 54) of the second arm (12), the sites passed through in sequence on the same arm being increasingly distant from the apex zone (3), and
- the second strand (22) passing through the braid (2) at at least two sites (60) of the second arm (12), the sites passed through in sequence being increasingly distant from the apex zone (3).

2. Device according to Claim 1, wherein the first strand (21) passes through the braid (2) at 2n+1 pairs of adjacent sites, alternately of the first arm (11) and of the second arm (12), with n being equal to 1 or 2.

3. Device according to Claim 1 or 2, wherein the first strand (21) passes through the braid (2) at two adjacent sites (51, 52) of the first arm (11), at two adjacent sites (53, 54) of the second arm (12), and at two adjacent sites (51, 52) of the first arm (11), in sequence and in this order, two sites (50) which are passed through in sequence on said first arm and said second arm being at a substantially identical distance from the apex zone (3).

4. Device according to one of the preceding claims, wherein the second strand (22) of the suture filament (20) passes through the braid (2) at two sites (61, 62) of the second arm (12), one in the vicinity of the apex zone (3) and the other in the vicinity of the distal portion (121) of said second arm.

5. Device according to the preceding claim, wherein the sites (53, 54) of the second arm (12) through which the first strand (21) passes are located between the two sites (61, 62) of said second arm through which the second strand (22) passes.

6. Device according to one of the preceding claims, wherein the free end (211) of the first strand (21) and the free end (221) of the second strand (22) each open out from a site (50) located at the distal portion (111, 121) of one of the arms of the anchor (1) in the space comprised between the two arms (11, 12) in a manner such that the distal ends of the first and second arms (11, 12) form fins (112, 122) spontaneously adopting a diverging orientation with respect to the axis of the anchor (1).

7. Device according to the preceding claim, wherein the distal ends of the first and second arms (11, 12) forming the fins (112, 122) are stiffened by thermosetting.

8. Device according to any one of the preceding claims, comprising a second suture filament (30), a median portion (33) of which separates a first and a second long strands (31, 32), each having a free end (311, 321), said strands passing through the braid (2) transversely from one side to the other at two sites (71, 72) of the apex zone (3) which are adjacent to each other, in a manner such that said median portion covers said apex zone and such that said first and second strands (31, 32) extend between the arms (11, 12) of the anchor (1).

9. Device according to the preceding claim, wherein the strands (31, 32) of the second suture filament (30) are interlaced with the portions of the first suture filament (20) passing transversely between the sites (50) of the first and of the second arms (11, 12).

10. Device according to any one of the preceding claims, wherein the anchor (1) is constituted by a braided tubular sheath, taken in a flattened configuration along its longitudinal axis.

11. Device according to the preceding claim, wherein the sheath is flattened while hot before cutting to a selected length comprised between 2 cm and 5 cm.

12. Device according to Claim 10 or Claim 11, wherein the sheath is formed from 4 to 6 textile fibers with a count of between 20 dtex and 200 dtex which are braided together.

13. Device according to any one of the preceding claims, wherein it is made of a resorbable or non-resorbable polymer material which is identical to or different for the anchor (1), for said at least one suture filament (20) and for the second suture filament (30) if it is present.

14. Device according to the preceding claim, wherein said polymeric material is selected from polyethylenes, polyethylene terephthalates and polylactic acids.

15. Surgical device for the repair of soft tissues according to any one of the preceding claims, having:
- an initial deployed configuration in which the arms (11, 12) of the anchor (1) are extended and the braid (2) is extended along its axis, said configuration having a first length and a first width allowing the device to be inserted into a hole in a human or animal bone, and
- a final compressed configuration obtained by pulling the strands (21, 22) of said at least one suture filament (20), and the strands (31, 32) of the second suture filament (30) if it is present, in which the arms (11, 12) of the anchor (1) are folded and the braid (2) is compacted along its axis, said configuration having a second length which is less than the first length and a second width which is greater than the first width, providing the device with its anchoring configuration in the hole.
